# EUROPÄISCHE PATENTSCHRIFT

(11) **EP 0 793 417 B1**
(45) Veröffentlichungstag und Bekanntmachung des Hinweises auf die Patenterteilung: **09.06.1999**
(21) Anmeldenummer: 95937819.1
(22) Anmeldetag: 23.10.1995
(51) Int. Cl.: A01N 35/02, C02F 1/50

(54) **ZUR ACROLEINFREISETZUNG BEFÄHIGTE ZUSAMMENSETZUNG UND DEREN VERWENDUNG**
ACROLEIN-RELEASING COMPOSITION AND USE THEREOF
COMPOSITION APTE A LA LIBERATION D'ACROLEINE ET SON UTILISATION

(30) Priorität: 21.11.1994 DE 4441315; 08.02.1995 DE 19504042
(43) Veröffentlichungstag der Anmeldung: 10.09.1997
(73) Patentinhaber: Degussa Aktiengesellschaft, 60311 Frankfurt (DE)
(72) Erfinder: WERLE, Peter, D-63571 Gelnhausen (DE); HELMLING, Oswald, D-63594 Hasselroth (DE); JAKOB, Harald, D-63594 Hasselroth (DE); TRAGESER, Martin, D-63571 Gelnhausen (DE)
(86) Internationale Anmeldenummer: EP9504142
(87) Internationale Veröffentlichungsnummer: WO9615668

(56) Entgegenhaltungen:
- EP-A- 0 493 658
- US-A- 3 690 857
- US-A- 4 851 583

## Beschreibung

Die Erfindung richtet sich auf eine zur Acroleinfreisetzung befähigte Zusammensetzung, welche durch Freisetzung von Acrolein in Gegenwart von Wasser eine biozide Wirksamkeit aufweist. Die Erfindung richtet sich auch auf die Verwendung der Zusammensetzung zur Bekämpfung mikrobieller, pflanzlicher und tierischer Schädlinge sowie ein Verfahren zur Durchführung.

In klimatisch warmen Zonen mit ausgedehnten landwirtschaftlich genutzten Feldern und Plantagen wird ein großes Netz an Bewässerungskanälen unterhalten. In diesen Kanälen kommt es leicht zur Veralgung und Verkrautung durch Wasserpflanzen. Die Pflanzen behindern die Fließgeschwindigkeit in den Kanälen und gefährden die störungsfreie Funktion der zum Betrieb notwendigen Pumpstationen. Aus diesem Grund ist es üblich, das Wasser in einem derartigen Bewässerungssystem mit einem Biozid zu dotieren. In der Praxis hat sich als Biozid Acrolein bewährt.

Acrolein bietet neben der bislang unübertroffenen Wirksamkeit auch den Vorteil, daß es in Wasser nach kurzer Zeit abgebaut ist. Damit ist Acrolein bis zur Bewässerung der Felder nicht mehr als Biozid wirksam, sondern zu pflanzenphysiologisch unbedenklichen Folgeprodukten abgebaut. Aufgrund der physikalischen und chemischen Eigenschaften des Acroleins ist aber seine Handhabung risikoreich: Acrolein ist giftig, stark inhalationstoxisch, stechend und tränenreizend und leicht entzündlich (Flammpunkt -29 °C, Siedepunkt 53 °C); durch Kontamination des Acroleins mit Verunreinigungen besteht ferner explosionsartige Polymerisationsgefahr.

In Anbetracht der dargestellten Risiken, welche sich sowohl beim Transport als auch bei der Handhabung von Acrolein ergeben, besteht ein Bedarf, die Dotierung mit einem vergleichbar wirksamen Biozid vorzunehmen, dessen Handhabung aber wesentlich weniger risikoreich ist.

Aus der US-PS 4,851,583 ist bekannt, das als Pestizid wirksame Acrolein durch Deacetalisierung von Acroleinacetalen, darunter auch cyclischen Acroleinacetalen, in Gegenwart eines stark sauren Ionenaustauschers als Katalysator zu gewinnen. Zur direkten Dotierung strömender Gewässer ist dieses Verfahren aber nicht geeignet, weil es rasch zur Verschmutzung beziehungsweise Inaktivierung des Ionenaustauschers käme. Die meisten Acroleinacetale weisen ferner nur eine sehr begrenzte Löslichkeit in Wasser auf, und zudem ist die Lösegeschwindigkeit niedrig: Beispielsweise werden zur Herstellung einer unter Praxisbedingungen nahezu gesättigten Lösung von 2-Vinyl-1,3-dioxolan (VDL) in Wasser (etwa 8 Gew.-%) bei intensiver Durchmischung etwa 10 Minuten benötigt; Die genannten Faktoren erschwerten bisher die Verwendung von Acroleinacetalen zur Freisetzung von Acrolein am Ort des Bedarfs, also unmittelbar an den Bewässerungskanälen, zumal dort elektrische Energie zwecks Intensivmischung nicht zur Verfügung steht.

Ein anderer Weg zur Dotierung von Wasser wird in der US-PS 5,183,944 beschrieben: Hiernach wird Acrolein durch Deacetalisierung von Acroleinacetalen in wäßriger Phase in Gegenwart eines stark sauren Katalysators gebildet. Zum Dotieren wäßriger Lösungen wird das während der Deacetalisierung in einem Reaktionsgefäß gebildete Acrolein ständig aus der wäßrigen Phase entfernt und in die zu dotierende wäßrige Lösung überführt wird. Die genannte Überführung erfolgt entweder mittels eines durch das Reaktionsgefäß geführten Gasstromes, der anschließend in die zu dotierende wäßrige Lösung geleitet wird, oder unter Verwendung einer Flüssigkeitsstrahlpumpe, deren Treibmittel die zu dotierende wäßrige Lösung ist. Dieses Verfahren eignet sich zwar zum Dotieren von beispielsweise Kühlwasserkreisläufen in Industrieanlagen, es läßt sich aber nicht zur Dotierung des Wassers in Bewässerungskanälen verwenden, weil am Ort der Dotierung im allgemeinen weder elektrische Energie zum Betreiben der Pumpen noch chemisch geschultes Fachpersonal zur Verfügung stehen.

In der noch nicht veröffentlichten DE-Patentanmeldung 43 26 575 wird ein Verfahren zum Dotieren strömender Gewässer mit Acrolein gelehrt, das die Nachteile der direkten Verwendung von Acrolein vermeidet und einfacher durchführbar ist als vorbekannte Verfahren unter Verwendung eines Acroleinacetals als Biozid-Percursor. Bei diesem Verfahren werden eine 25 bis 95 gew.-%ige Lösung eines Acroleinacetals in einem organischen Lösungsmittel und eine 3 bis 30 gew.-%ige wäßrige Mineralsäurelösung zunächst in festgelegtem Mengenverhältnis in eine Mischkammer eingebracht, das Reaktionsgemisch anschließend durch einen speziell konstruierten Deacetalisierungsreaktor geleitet und dann in das zu dotierende Gewässer überführt.

Obwohl das zuvor gewürdigte Verfahren eine Problemlösung darstellt, weist es doch noch einige Nachteile auf: Zur Durchführung sind ein spezieller Deacetalisierungsreaktor und außer einem druckfesten Vorratsbehälter für das Acroleinacetal zusätzlich ein solcher für eine wäßrige Mineralsäure erforderlich, wodurch sich der Aufwand erhöht; auch die Verhältnisregelung zwischen dem Acroleinacetal und der wäßrigen Säure mittels Lochblenden ist nicht unproblematisch.

Die Aufgabe der vorliegenden Erfindung besteht somit darin, ein verbessertes Verfahren aufzuzeigen, womit mikrobielle, pflanzliche und tierische Schädlinge mittels Acrolein bekämpft werden können, ohne jedoch Acrolein selbst an den Bedarfsort transportieren und dort handhaben zu müssen. Das Verfahren sollte einfach handhabbar und nicht auf elektrische Energiezufuhr, etwa zum Betreiben von Pumpen, Rühr- und Heizvorrichtungen, angewiesen sein. Eine weitere Aufgabe richtet sich auch darauf, eine sicher handhabbare, zur Acroleinfreisetzung befähigte Zusammensetzung zur Verfügung zu stellen, bei deren Verwendung sich die Bereitstellung einer zweiten Chemikalie und deren Zumischung zur Zusammensetzung am Ort der Verwendung zwecks Acroleinfreisetzung erübrigen. Schließlich sollte sich die Freisetzung von Acrolein aus der Zusammensetzung ohne das Erfordernis eines speziellen Reaktors durchführen lassen.

Die Aufgabe wird durch eine zur Acroleinfreisetzung befähigte Zusammensetzung gelöst, welche gekennzeichnet ist (i) durch einen Gehalt an einem Acetal des Acroleins mit einem Alkohol mit 1 bis 6 C-Atomen und 1 bis 4 Hydroxlgruppen, (ii) einen Gehalt an einer im Acetal zumindest teilweise löslichen und damit chemisch verträglichen Säure mit einem pKₛ-Wert von kleiner 4 und (iii) Abwesenheit von Wasser.

Die Unteransprüche richten sich auf bevorzugte Ausführungsformen der Zusammensetzung.

Es wurde gefunden, daß ein anspruchsgemäßes Acroleinacetal oder ein Gemisch solcher Acroleinacetale in Abwesenheit von Wasser im Gemisch mit einer oder mehreren anspruchsgemäßen Säuren ausreichend lagerstabil ist, es also zu keiner Deacetalisierung kommt. Die erfindungsgemäße Zusammensetzung ist damit in feuchtigkeitsdichten Gebinden transport- und lagerfähig und weist nicht die vom Acrolein bekannten Handhabungsprobleme auf. Erst beim Kontaktieren der Zusammensetzung mit Wasser kommt es zur Deacetalisierung des Acetals und Freisetzung des biozid wirksamen Acroleins. Die in der Zusammensetzung enthaltene Säure wirkt als Deacetalisierungskatalysator.

Bei den erfindungsgemäß in der Zusammensetzung enthaltenen Acroleinacetalen handelt es sich um solche mit (i) einwertigen Alkoholen, insbesondere Methanol, Ethanol, n- und iso-Propanol, (ii) zweiwertigen Alkoholen, insbesondere Ethylenglykol, 1,2- und 1,3-Propylenglykol, (iii) dreiwertigen Alkoholen, insbesondere Glycerin, Trimethylolpropan und 1,2,6-Hexantriol, und (iv) Pentaerythrit. Besonders bevorzugte Acroleinacetale sind solche mit einer 2-Vinyl-1,3-dioxolan- oder 2-Vinyl-1,3-dioxanstruktur, insbesondere 2-Vinyl-1,3-dioxolan und 2-Vinyl-4-hydroxymethyl-1,3-dioxolan, leicht erhältlich aus Acrolein und Ethylenglykol beziehungsweise Glycerin. Acroleinacetale mit längeren aliphatischen Gruppen werden wegen ihrer geringen Löslichkeit in Wasser weniger bevorzugt. Sofern das Acroleinacetal noch freie Hydroxylgruppen enthält, ist im Einzelfall zu prüfen, ob die erforderliche chemische Stabilität in Gegenwart der anspruchsgemäßen Säure gegeben ist.

An die in der Zusammensetzung enthaltenen Säuren werden eine Reihe von Anforderungen gestellt: Sie müssen in ausreichender Menge im Acroleinacetal löslich und wasserfrei sein und einen pKₛ-Wert von kleiner 4, vorzugsweise kleiner 3, aufweisen. Besonders bevorzugt wird eine Säure oder Säuregemisch mit einem pKₛ-Wert im Bereich von 1 bis 2,5. Die Säure und das Acroleinacetal müssen in Abwesenheit von Feuchtigkeit im Gemisch miteinander chemisch beständig sein - diese Beständigkeit wird der Fachmann durch orientierende Versuche ermitteln, um zu einer lagerstabilen erfindungsgemäßen Zusammensetzung zu gelangen. Vorzugsweise soll die Säure auch biologisch abbaubar und möglichst nicht toxisch sein.

Unter den Carbonsäuren sind Ameisensäure mit einem pKₛ-Wert von knapp unter 4 und Trihydroxybenzoesäure wirksam, jedoch weisen viele andere Carbonsäuren, etwa Essigsäure, eine zu geringe Säurestärke auf, um nach dem Kontaktieren der Zusammensetzung mit Wasser eine ausreichend rasche Hydrolyse des Acroleinacetals zu ermöglichen. Als überraschend gut wirksam haben sich etliche Di- und Polycarbonsäuren, sofern ihr pKₛ-Wert unter 4 liegt und eine ausreichende Wasserlöslichkeit gegeben ist, erwiesen. Beispiel hierfür sind: Oxalsäure, Maleinsäure, Fumarsäure Dihydroxymalein- beziehungsweise - fumarsäure, Mellitsäure und Pyromellitsäure. Verwendbar sind auch α-halogenierte niedere Carbonsäuren, wie Chlor- oder Bromessigsäure, verwendbar; aus ökologischen Gründen weniger bevorzugt sind fluorierte Carbonsäuren. Unter den Carbonsäuren werden wasserfreie Oxalsäure und Maleinsäure oder Fumarsäure besonders bevorzugt. Geeignete Säuren sind auch saure Phosphorsäureester, niedere Phosphonsäuren, etwa Hydroxymethanphosphonsäure, saure Phosphonsäureester und niedere Sulfonsäuren, wie Methansulfonsäure, und niedere Schwefelsäuremonoalkylester. Besonders geeignet sind Phosphorsäure-monoalkylester oder -dialkylester oder Gemische dieser Ester, wobei Alkyl bevorzugt für Methyl, Ethyl, n-Propyl oder iso-Propyl steht. Auch saure Phosphorsäureester aus der Umsetzung von Phosphorsäure mit einem Diol, etwa Ethylenglykol oder 1,3-Propandiol, sind geeignet. Anorganische Säuren, wie H₂SO₄, H₃PO₄ sowie HNO₃, sind wegen ihrer chemischen Unverträglichkeit mit den Acroleinacetalen nicht geeignet.

Das Acroleinacetal oder Acroleinacetalgemisch und die Säure oder Säuregemisch können an sich in beliebigem Molverhältnis in der Zusammensetzung anwesend sein, zweckmäßigerweise liegt aber das Molverhältnis im Bereich von 50 zu 1 bis 1 zu 1, insbesondere 50 zu 1 bis 10 zu 1. Je höher die Säurekonzentration in der Zusammensetzung ist, desto rascher erfolgt die Deacetalisierung des Acetals nach Zugabe von Wasser. Der Gehalt an Acroleinacetal in der Zusammensetzung wird, um mit einer gegebenen Menge Zusammensetzung eine möglichst hohe biozide Wirksamkeit erzielen zu können, möglichst hoch sein. Eine besonders bevorzugte Zusammensetzung enthält ein bevorzugtes Acroleinacetal oder Gemisch mehrerer Acetale und eine oder mehrere der bevorzugten Dicarbonsäuren oder sauren Phosphorsäureester im Gewichtsverhältnis im Bereich von 97 zu 3 bis 80 zu 20, inbesondere im Bereich von 95 zu 5 bis 90 zu 10.

Zweckmäßigerweise enthält die erfindungsgemäße Zusammensetzung zusätzlich ein oder mehrere, in Gegenwart der Säure und des Acroleinacetals stabile Tenside. Die Einsatzmenge an Tensid liegt üblicherweise zwischen 5 und 0,05 Gew.-%, bezogen auf die Zusammensetzung, meistens ist aber eine Menge zwischen 0,2 bis 1 Gew.-% ausreichend. Besondes geeignete Tenside sind Ethoxylierungsprodukte von Fettalkoholen, Alkylphenolen, Fettsäuren, Fettamiden und Glycerinmono- und -difettsäureestern. Das Tensid muß in der Zusammensetzung ausreichend löslich und zudem in der Lage sein, die Zusammensetzung nach Kontaktieren mit Wasser zu emulgieren. In Abwesenheit eines Tensids oder zu geringer Konzentration hieran kann es bei wenig wasserlöslichen Acroleinacetalen, etwa 2-Vinyl-1,3-dioxolan, zu einer Entmischung kommen, so daß die Deacetalisierung unvollständig oder verzögert abläuft. In Anwesenheit einer ausreichenden Menge eines wirksamen Tensids löst sich die erfindungsgemäße Zusammensetzung innerhalb weniger Sekunden klar auf, und die Deacetalisierung läuft auch bei etwa 20 °C innerhalb weniger Minuten praktisch quantitativ ab.

Eine besonders vorteilhafte Zusammensetzung besteht im wesentlichen aus 80 bis 97 Gew.-% 2-Vinyl-1,3-dioxolan, 2 bis 20 Gew.-% Dicarbonsäure aus der Reihe wasserfreier Oxalsäure, Maleinsäure und Fumarsäure, insbesondere wasserfreie Oxalsäure, oder Phosphorsäuremono- und/oder -di(C₁- bis C₃)-alkylester und 0,1 bis 5 Gew.-%, vorzugsweise 0,2 bis 1 Gew.-%, eines nichtionischen Tensids auf der Basis eines Ethoxylierungsproduktes der zuvor genannten Stoffklassen; besonders bevorzugt enthält die Zusammensetzung als Tensid ein Polyoxyethylenaddukt eines Glycerinmonofettsäureesters.

Außer den erfindungswesentlichen Komponenten und dem vorzugsweise zusätzlich anwesenden Tensid kann die Zusammensetzung weitere Zusatzstoffe enthalten, soweit sie in der Zusammensetzung stabil sind. Mögliche weitere Zusatzstoffe sind beispielsweise andere Biozide und wasserlösliche organische Lösungsmittel, wie niedere Alkohole.

Die erfindungsgemäßen Zusammensetzungen lassen sich durch einfaches Zusammenmischen der Komponenten herstellen und sind im allgemeinen klare Lösungen. Die Verträglichkeit der Komponenten untereinander wird zuvor durch Lagertests sichergestellt.

Die erfindungsgemäße Zusammensetzung läßt sich, da beim Kontakt mit Wasser das hochwirksame Biozid Acrolein gebildet wird, als Mittel zur Bekämpfung mikrobieller, pflanzlicher und tierischer Schädlinge verwenden. Unter den Schädlingen sind insbesondere Bakterien und Pilze, Algen und Wasserkräuter, Insekten, Würmer und Nager zu verstehen.

Zur Bekämpfung mikrobieller, pflanzlicher und tierischer Schädlinge mittels Acrolein wird eine erfindungsgemäße Zusammensetzung entweder (i) unmittelbar mit einem die zu bekämpfenden Schädlinge enthaltenden feuchten Medium in Kontakt gebracht oder (ii) im Gewichtsverhältnis von 1 zu 1 bis 1 zu 50 mit Wasser gemischt, das Reaktionsgemisch nach einer zur weitgehenden Deacetalisierung des in der Zusammensetzung enthaltenden Acroleinacetals erforderlichen Reaktionszeit unmittelbar oder nach weiterer Verdünnung mit Wasser in das die zu bekämpfenden Schädlinge enthaltende Medium eingebracht.

Das Verhältnis Acroleinacetal zu Wasser in der Verfahrensalternative (ii) liegt zweckmäßigerweise in einem solchen Bereich, daß während der Deacetalisierung ein pH-Wert um/unter 2 während einiger Minuten aufrechterhalten wird. Im allgemeinen liegt das Verhältnis zwischen 1 zu 1 und 1 zu 50, vorzugsweise zwischen 1 zu 5 und 1 zu 20.

Zum Zwecke einer raschen Auflösung der Zusammensetzung in Wasser wird diese vorzugsweise mit einer Ein- oder besser Mehrfachsprühdüse in Wasser eingedüst, so daß sofort feinste und damit gut emulgierbare und rasch lösliche Tröpfchen resultieren. Das Eindüsen läßt sich beispielsweise unter Verwendung entsprechender Düsen und einer Druckgasflasche bewirken. Bei der Verfahrensalternative (i) wird Acrolein dem Feuchtegehalt des Mediums entsprechend langsamer freigesetzt. Die Verfahrensalternative (i) läßt sich beispielsweise zur Bekämpfung von Nematoden oder Nagern im Erdboden anwenden.

Zur Bekämpfung von aquatischen Schädlingen, etwa Algen und Kräutern in Bewässerungskanälen, eignet sich besonders die Alternative (ii). Hierbei wird das deacetalisierte Gemisch kontinuierlich oder periodisch in einer solchen Menge in das die Schädlinge enthaltende Wasser eingeleitet, daß darin ein Acroleingehalt im Bereich von 0,1 bis 20 ppm Acrolein aufrechterhalten wird.

Vorteile der erfindungsgemäßen Zusammensetzung sind ihre leichte Herstellbarkeit, ihre gute Lager- und Handhabbarkeit und ihre unmittelbare Anwendbarkeit zur Bekämpfung von Schädlingen. Da die Zusammensetzung selbst den Deacetalisierungskatalysator enthält, muß ein solcher nicht am Ort der Verwendung der Acroleinacetal enthaltenden Zusammensetzung in dosierter Form zugemischt werden. Aufgrund der ausgewählten Bestandteile der Zusammensetzung sind weder eine aufwendige Vorrichtung noch ein spezieller Deacetalisierungsreaktor erforderlich, um aus dem Acroleinacetal Acrolein freisetzen zu können. Das Verfahren zur Bekämpfung von bakteriellen, pflanzlichen und tierischen Schädlingen gestaltet sich damit einfach, erfordert keine elektrische Energie und kann von wenig geschultem Personal durchgeführt werden.

### Beispiel 1: Herstellung der Zusammensetzung und Freisetzung von Acrolein

95 g 2-Vinyl-1,3-dioxolan (VDL), 5 g einer 60:40 Mischung aus Monoisopropyl- und Diisopropylphosphorsäureester sowie 1 g Tensid auf der Basis Polyoxyethylen-glycerin-monooleat (Tegotens® 02 der Firma Th. Goldschmidt AG) werden gemischt. Die Zusammensetzung ist eine klare Lösung.

Die so erhaltene Zusammensetzung wird mittels einer Sprühdüse mit einem Druck aus einer N₂-Druckgasflasche von 3 bar in 1000 ml Wasser eingedüst. Die zunächst gebildete Emulsion wird in wenigen Sekunden eine klare Lösung. Nach 10 Minuten Verweilzeit haben sich 99,6 % des im VDL gebundenen Acroleins freigesetzt (gaschromatographische Bestimmung).

Durch Zugabe des freigesetztes Acrolein enthaltenden wäßrigen Konzentrats zu aquatische Schädlinge enthaltendem Wasser im Verhältis von beispielsweise 1 zu 5000 stellt sich eine Acroleinkonzentration von etwa 10,6 ppm (parts per million) ein, welche zur Bekämpfung der Schädlinge geeignet ist.

### Beispiel 2

Zu einer Mischung aus 470,2 g VDL und 4,7 g Tensid gemäß Beispiel 1 (Tegotens® 02) werden unter Rühren 25 g wasserfreie Oxalsäure gegeben. Nach ca. 10 min erhält man eine praktisch farblose, homogene Lösung. Die Lösung "L" ist lagerstabil.

Untersuchung der Acroleinfreisetzung:
Die Lösung "L" wird in dem in der Tabelle angegebenen Volumenverhältnis in Wasser eingetragen; die Mischung wird gerührt, wobei Hydrolyse erfolgt und Acrolein freigesetzt wird. Die Reaktion wird durch Zugabe von Natronlauge (End-pH: 6-7) gestoppt; das gebildete Acrolein und Ethylenglykol sowie der Restgehalt an VDL werden gaschromatographisch gemessen. Es ergibt sich eine Umsetzung des VDL gemäß Tabelle.

| Volumenverhältnis "L" zu Wasser | Umsetzung von VDL (Hydrolyserate) in % nach | | |
|---|---|---|---|
| | 2 min | 4 min | 8 min |
| 1 : 5 | 85,9 | 98,6 | 99,5 |
| 1 : 10 | 91,0 | 99,2 | 99,7 |
| 1 : 20 | 71,7 | 92,9 | 99,6 |

### Beispiel 3

In einer Mischung aus 188,2 g VDL und 0,9 g Tensid gemäß Beispiel 1 (Tegotens® 02) werden unter Rühren 10 g wasserfreie Maleinsäure bei 35 °C gelöst. 10 ml dieser Lösung werden in 60 ml Wasser eingetragen; die Mischung wird 6 min gerührt, die Reaktion mittels NaOH gestoppt und der Restgehalt an nichthydrolysiertem VDL ermittelt. Es ergibt sich eine Umsetzung von 98,2 %.

### Beispiel 4: Verfahren zur Verhinderung einer Veralgung und Verkrautung strömender Gewässer

Eine erfindungsgemäße Zusammensetzung wird in einem mit Steigrohr versehenen feuchtigkeitsdicht verschlossenen Behälter zum Applikationsort gebracht. Durch Anlegen eines Drucks von 3 bar aus einer N₂-Druckgasflasche auf den Behälter wird die aus dem Steigrohr austretende Zusammensetzung über einen druckfesten Schlauch in das zu dotierende Wasser geleitet. Am Ende dieses Schlauches befindet sich eine Düse, die es ermöglicht, eine feine Verteilung der Zusammensetzung in einer zunächst begrenzten Menge des zu dotierenden Wassers zu erzielen. Um die für die Acetalspaltung notwendigen Bedingungen hinsichtlich Zeit und Acidität zu erhalten, wird die sich bildende Lösung zunächst nur soweit verdünnt, daß über einen Zeitraum von etwa 5 bis 10 Minuten ein pH-Wert von etwa 1,5 bis 2 aufrechterhalten wird. Nach ausreichender Deacetalisierung wird das Acrolein enthaltende deacetalisierte wäßrige Konzentrat mit einer ausreichenden Menge des zu dotierenden Wassers verdünnt, um zur gewünschten Anwendungskonzentration zu gelangen.

Eine geeignete Maßnahme zur Einhaltung optimaler Deacetalisierungsbedingungen besteht darin, daß das Eindüsen in einen mit einer definierten Menge Wasser gefüllten Behälter erfolgt; dieser Behälter befindet sich zweckmäßigerweise selber in dem zu dotierenden Wasser. Nach Ablauf der Verweilzeit wird der Behälter geöffnet und das freigesetzte Acrolein in den Kanal geschwemmt. Alternativ kann die Eindüsung in eine in dem zu dotierenden Wasser befindliche wasserdurchströmte Röhre (oder Schlauch) erfolgen, die infolge ihrer Länge und ihres Querschnitts bei der vorhandenen Fließgeschwindigkeit nur eine begrenzte Menge Wasser durchläßt und eine ausreichende Verweilzeit vor weiterem Verdünnen gewährleistet.

## Patentansprüche

1. Zur Acroleinfreisetzung befähigte Zusammensetzung, gekennzeichnet durch (i) einen Gehalt an einem Acetal des Acroleins mit einem Alkohol mit 1 bis 6 C-Atomen und 1 bis 4 Hydroxlgruppen, (ii) einen Gehalt an einer im Acetal zumindest teilweise löslichen und damit chemisch verträglichen Säure mit einem pKₛ-Wert von kleiner 4 und (iii) Abwesenheit von Wasser.

2. Zusammensetzung nach Anspruch 1,
dadurch gekennzeichnet,
daß sie zusätzlich ein Tensid enthält.

3. Zusammensetzung nach Anspruch 1 oder 2,
dadurch gekennzeichnet,
daß sie als Acroleinacetal 2-Vinyl-1,3-dioxolan enthält.

4. Zusammensetzung nach einem der Ansprüche 1 bis 3,
dadurch gekennzeichnet,
daß der pKₛ-Wert der Säure kleiner 3 ist und vorzugsweise im Bereich zwischen 1 und 2,5 liegt.

5. Zusammensetzung nach einem der Ansprüche 1 bis 4,
dadurch gekennzeichnet,
daß sie als Säure eine organische Di- oder Polycarbonsäure enthält.

6. Zusammensetzung nach Anspruch 5,
dadurch gekennzeichnet,
daß sie als Säure Oxalsäure, Fumarsäure oder Maleinsäure enthält.

7. Zusammensetzung nach einem der Ansprüche 1 bis 6,
dadurch gekennzeichnet,
daß sie als Säure einen Phosphorsäuremono- und/oder -dialkylester eines Alkohols mit 1 bis 3 C-Atomen und 1 oder 2 Hydroxylgruppen enthält.

8. Zusammensetzung nach einem der Ansprüche 1 bis 7,
dadurch gekennzeichnet,
daß sie das Acroleinacetal und die Säure im Molverhältnis 50 zu 1 bis 1 zu 1 enthält.

9. Zusammensetzung nach einem der Ansprüche 1 bis 8,
dadurch gekennzeichnet,
daß sie als Tensid ein Ethoxylierungsprodukt eines Fettalkohols, einer Fettsäure, eines Fettamids oder eines Glycerinmono- oder -difettsäureesters enthält.

10. Zusammensetzung nach einem der Ansprüche 1 bis 9,
dadurch gekennzeichnet,
daß sie im wesentlichen aus 80 bis 97 Gew.-% 2-Vinyl-1,3-dioxolan, 2 bis 20 Gew.-% Phosphorsäuremono- und/oder -di(C₁- bis C₃-)alkylester und 0,1 bis 5 Gew.-% Tensid gemäß Anspruch 9 besteht.

11. Zusammensetzung nach einem der Ansprüche 1 bis 9,
dadurch gekennzeichnet,
daß sie im wesentlichen aus 80 bis 97 Gew.-% Vinyldioxolan, 2 bis 20 Gew.-% wasserfreier Oxalsäure und 0,1 bis 5 Gew.-% Tensid gemäß Anspruch 9 besteht.

12. Verwendung der Zusammensetzung gemäß einem der Ansprüche 1 bis 11 als Mittel zur Bekämpfung mikrobieller, pflanzlicher und tierischer Schädlinge.

13. Verfahren zur Bekämpfung mikrobieller, pflanzlicher und tierischer Schädlinge mittels Acrolein,
dadurch gekennzeichnet,
daß man eine Zusammensetzung gemäß einem der Ansprüche 1 bis 11 entweder unmittelbar mit einem die zu bekämpfenden Schädlinge enthaltenden feuchten Medium in Kontakt bringt oder die Zusammensetzung im Gewichtsverhältnis von 1:1 bis 1:50 mit Wasser mischt, das Reaktionsgemisch nach einer zur weitgehenden Deacetalisierung des in der Zusammensetzung enthaltenden Acroleinacetals erforderlichen Reaktionszeit unmittelbar oder nach weiterer Verdünnung mit Wasser in das die zu bekämpfenden Schädlinge enthaltende Medium einbringt.

14. Verfahren nach Anspruch 13 zur Verhinderung einer Veralgung und Verkrautung aquatischer Systeme,
dadurch gekennzeichnet,
daß man in das aquatische System das deacetalisierte Reaktionsgemisch kontinuierlich oder periodisch in einer solchen Menge einleitet, daß darin ein Acroleingehalt im Bereich von 0,1 bis 20 ppm aufrechterhalten wird.

## Claims

1. Composition capable of releasing acrolein, characterized by (i) a content of an acetal of acrolein with an alcohol having 1 to 6 C atoms and 1 to 4 hydroxyl groups, (ii) a content of an acid at least partly soluble in the acetal and chemically compatible therewith, having a pKₐ value of less than 4 and (iii) absence of water.

2. Composition according to claim 1,
characterised in that
it contains in addition a surfactant.

3. Composition according to claim 1 or 2,
characterised in that
it contains 2-vinyl-1,3-dioxolane as acrolein acetal.

4. Composition according to one of claims 1 to 3,
characterised in that
the pKₐ value of the acid is less than 3 and is preferably within the range between 1 and 2.5.

5. Composition according to one of claims 1 to 4,
characterised in that
it contains as acid an organic dicarboxylic or polycarboxylic acid.

6. Composition according to claim 5,
characterised in that
it contains as acid oxalic acid, fumaric acid or maleic acid.

7. Composition according to one of claims 1 to 6,
characterised in that
it contains as acid a monoalkyl phosphate ester and/or dialkyl phosphate ester of an alcohol having 1 to 3 C atoms and 1 or 2 hydroxyl groups.

8. Composition according to one of claims 1 to 7,
characterised in that
it contains the acrolein acetal and the acid in the molar ratio of 50 to 1 up to 1 to 1.

9. Composition according to one of claims 1 to 8,
characterised in that
it contains as surfactant an ethoxylation product of a fatty alcohol, of a fatty acid, of a fatty amide or of a mono- or diglyceride fatty acid ester.

10. Composition according to one of claims 1 to 9,
characterised in that
it consists substantially of from 80 to 97 wt.% of 2-vinyl-1,3-dioxolane, from 2 to 20 wt.% of monoalkyl phosphate esters and/or di(C₁- to C₃-)alkyl phosphate esters and from 0.1 to 5 wt.% of surfactant according to claim 9.

11. Composition according to one of claims 1 to 9,
characterised in that
it consists substantially of from 80 to 97 wt.% of vinyldioxolane, from 2 to 20 wt.% of anhydrous oxalic acid and from 0.1 to 5 wt.% of surfactant according to claim 9.

12. Use of the composition according to one of claims 1 to 11 as an agent for the control of microbial, vegetable and animal pests.

13. Method for the control of microbial, vegetable and animal pests by means of acrolein,
characterised in that
a composition according to one of claims 1 to 11 is either brought directly into contact with a damp medium containing the pests to be controlled or the composition is mixed with water in the weight ratio of 1:1 up to 1:50; the reaction mixture, after a reaction time necessary for the extensive deacetalation of the acrolein acetal contained in the composition, is introduced into the medium containing the pests to be controlled, directly or after further dilution with water.

14. Method according to claim 13 for the prevention of algal growth and excessive plant growth in aquatic systems,
characterised in that
the deacetalated reaction mixture is introduced continuously or periodically into the aquatic system in a quantity such that an acrolein content within the range of from 0.1 to 20 ppm is maintained therein.

## Revendications

1. Composition apte à la libération de l'acroléine, caractérisée par (i) une teneur en un acétal de l'acroléine avec un alcool ayant 1 à 6 atomes de carbone et 1 à 4 groupes hydroxyles, (ii) une teneur en un acide au moins partiellement soluble dans l'acétal et chimiquement compatible avec lui ayant un pKₛ inférieur à 4 et (iii) par l'absence d'eau.

2. Composition selon la revendication 1,
caractérisée en ce qu'
elle contient en outre un agent tensioactif.

3. Composition selon la revendication 1 ou 2,
caractérisée en ce qu'
elle contient comme acroléine-acétal le 2-vinyl-1,3-dioxolane.

4. Composition selon l'une des revendications 1 à 3,
caractérisée en ce que
le Pkₛ de l'acide est inférieur à 3 et se situe de préférence dans un intervalle compris entre 1 et 2,5.

5. Composition selon l'une des revendications 1 à 4,
caractérisée en ce qu'
elle contient comme acide un acide di- ou percarboxylique organique.

6. Composition selon la revendication 5,
caractérisé en ce qu'
elle contient comme acide l'acide oxalique, l'acide fumarique ou l'acide maléique.

7. Composition selon l'une des revendications 1 à 6,
caractérisée en ce qu'
elle contient comme acide un mono- et/ou dialkylester d'acide phosphorique d'un alcool ayant 1 à 3 atomes de carbone et un ou deux groupes hydroxyle.

8. Composition selon l'une des revendications 1 à 7,
caractérisée en ce qu'
elle contient l'acroléine-acétal et l'acide dans un rapport molaire de 50:1 à 20:1.

9. Composition selon l'une des revendications 1 à 8,
caractérisée en ce qu'
elle contient comme agent tensioactif un produit d'éthoxylation d'un alcool gras, d'un acide gras, d'un amide gras ou d'un mono- ou diester d'acide gras de glycérine.

10. Composition selon l'une des revendications 1 à 9,
caractérisée en ce qu'
elle se compose essentiellement de 80 à 97 % en poids de 2-vinyl-1,3-dioxolane, de 2 à 20 % en poids de mono- et/ou dialkyl en C₁ à C₃-ester d'acide phosphorique et de 0,1 à 5 % en poids d'agent tensioactif selon la revendication 9.

11. Composition selon l'une des revendications 1 à 9,
caractérisée en ce qu'
elle se compose essentiellement de 80 à 97 % en poids de vinyldioxolane, de 2 à 20 % en poids d'acide oxalique anhydre et de 0,1 à 5 % en poids d'agent tensioactif selon la revendication 9.

12. Utilisation de la composition selon l'une des revendications 1 à 11, comme agent pour combattre les parasites microbiens, végétaux et animaux.

13. Procédé pour combattre les parasites microbiens, végétaux et animaux au moyen d'acroléine,
caractérisé en ce qu'
on met en contact une composition selon l'une des revendications 1 à 11 soit immédiatement avec un milieu humide contenant les parasites à combattre, soit qu'on mélange la composition avec l'eau dans un rapport pondéral de 1:1 à 1:50, en ce qu'on met le mélange réactionnel après large désacétylation de l'acroléine acétal contenu au bout du temps nécessaire à une large désacétylation de l'acroléine acétal contenu dans la composition immédiatement ou après une dilution plus poussée avec de l'eau dans le milieu contenant les parasites à combattre.

14. Procédé selon la revendication 13 pour empêcher une pollution par les algues et les mauvaises herbes de systèmes aquatiques,
caractérisé en ce qu'
on introduit dans le système aquatique le mélange réactionnel désacétylisé de façon continue ou périodique dans une quantité telle qu'on y maintient une teneur en acroléine comprise entre 0,1 et 20 ppm.
